# EUROPÄISCHE PATENTANMELDUNG

(11) **EP 1 762 266 A1**
(43) Veröffentlichungstag der Anmeldung: **14.03.2007**
(21) Anmeldenummer: 06076543.5
(22) Anmeldetag: 07.08.2006
(51) Int. Cl.: A61N 1/05, H01R 4/28

(54) **Einschraubelektrodensonde zur kardiologischen Anwendung**

(30) Priorität: 07.09.2005 DE 102005042369
(71) Anmelder: BIOTRONIK CRM Patent AG, 6341 Baar (CH)
(72) Erfinder: Feldmann, Jörg, 12683 Berlin (DE); Kolberg, Gernot, 12043 Berlin (DE); Lenski, Hartmut, 15806 Glienicke (DE)
(74) Vertreter: Lindner-Vogt, Karin L.

(57) **Zusammenfassung**

Eine Einschraubelektrodensonde zur kardiologischen Anwendung umfasst einen langgestreckten Elektrodenkörper (1), eine darin verlaufende Zuleitung (10) und einen Elektrodenkopf (3) am distalen Ende (2). Letzterer ist mit einem Gehäuse (5), einer darin drehbar und axial verschiebbar gelagerten Welle (6) und einer korkenzieherartigen Einschraubelektrode (4) an der Welle (6) versehen. Zwischen diesen Bauteilen ist eine Kontaktfeder in Form einer im Wesentlichen ebenen Spiralfeder (11) angeordnet, die mit ihren Schenkelenden (12, 13) unter Ausübung einer radial gerichteten Federkraft (F) gleitend mit dem Gehäuse (5) bzw. der Welle (6) in Kontakt steht.

## Beschreibung

Die Erfindung betrifft eine Einschraubelektrodensonde zur kardiologischen Anwendung mit den im Oberbegriff des Patentanspruches 1 angegebenen Merkmalen.

Derartige Elektrodensonden sind durch offenkundige Vorbenutzungen bekannt oder auch aus der US 2002/0188340 A1 oder der US 2003/0144722 A1 entnehmbar. Sie weisen einen langgestreckten Elektrodenkörper aus einem elektrisch isolierenden Material, eine im Elektrodenkörper verlaufende, drehbare, elektrische Zuleitung und einen am distalen Ende des Elektrodenkörpers angeordneten Elektrodenkopf auf. Letzterer ist mit einem Gehäuse versehen, aus dem eine korkenzieherartige Einschraubelektrode durch Drehung um ihre Wendelachse unter entsprechender axialer Verschiebung in kardiales Gewebe einschraubbar ist. Dazu sitzt die Einschraubelektrode koaxial auf einer Welle, die wiederum in axialer Verlängerung mit der im Elektrodenkörper verlaufenden Zuleitung - in der Regel ein gewendelter Draht - für die Elektrode verbunden ist. Über die Zuleitung kann auf die Welle ein Drehmoment übertragen werden, durch einen entsprechenden Steigungsgeber wird dann die Welle um ihre Längsachse rotiert und axial verschoben, um die Einschraubelektrode aktiv in das kardiale Gewebe einzuschrauben bzw. daraus wieder zurückzuholen.

Je nach Anwendungsfall kann es aus signal- oder stimulationstechnischen Gründen notwendig sein, zwischen dem aus Metall bestehenden, elektrisch leitfähigen Gehäuse und der Einschraubelektrode selbst eine elektrische Verbindung herzustellen, damit beide Elemente auf dem gleichen elektrischen Potenzial liegen. Dies wird herkömmlicherweise durch eine Kontaktfeder realisiert, für die verschiedene Ausgestaltungen und Einbaumöglichkeiten durch offenkundige Vorbenutzung bekannt sind. Grundsätzlich ist dabei im Zusammenhang mit der aktiven Fixierung und der damit verbundenen Rotation der Einschraubelektrode mit ihrer Welle und der gleichzeitigen Axialverschiebung darauf zu achten, dass die Kontaktfeder in der Lage ist, neben der axialen Bewegung von Welle und Einschraubelektrode auch radiale Verschiebungen zu kompensieren. Dazu ist es bekannt, eine Art Uhrenfeder, wie sie beispielsweise für die Unruh einer Uhr eingesetzt wird, mit beiden Enden an der Welle bzw. dem Gehäuse anzuschweißen. Aufgrund der sehr filigranen Ausführung dieser Feder ist dieser Montageschritt relativ aufwändig und sensibel, was die Zuverlässigkeit der Kontaktverbindungen angeht. Ferner beansprucht diese Art von Federn einen relativ großen Bauraum, da der Verschiebungsweg, den die fixen Anschlusspunkte der Uhrenfeder an Gehäuse und Welle relativ zueinander zurücklegen müssen, vergleichsweise groß, nämlich beispielsweise zwei Umdrehungen bei einem axialen Versatz von 2 mm ist. Dieser Weg muss durch die Länge der Federschenkel bereitgestellt werden, ohne bei der Rotation und Axialverschiebung der Einschraubelektrode eine nennenswerte Gegenspannung aufzubauen.

Eine weitere bekannte Möglichkeit liegt in dem Einsetzen einer Schraubendruckfeder zwischen dem Gehäuse und einem entsprechenden Anschlag an der Welle bzw. Einschraubelektrode. Eine derartige Anordnung beansprucht allerdings in axialer Richtung einen relativ großen Bauraum. Darüber hinaus wirkt die Federkraft der Schraubendruckfeder dem Einschieben der Einschraubelektrode beim aktiven Zurückziehen in das Gehäuse entgegen, was für die Elektrodenbetätigung hinderlich ist.

Schließlich ist bereits versucht worden, eine Art Schenkelfeder zwischen der Elektrodenwelle und dem Gehäuse zur Verbindung einzusetzen. Wegen des geringen zur Verfügung stehenden Bauraumes und der entsprechend filigranen Dimensionierung hat diese Kontaktfeder jedoch eine sehr geringe Kontaktkraft, so dass eine Kontaktstabilität insbesondere bei den für Einschraubelektroden üblichen Radialschwankungen der Wendel nicht gewährleistet werden kann.

Ausgehend von den geschilderten Problemen liegt der Erfindung die Aufgabe zugrunde, eine Einschraubelektrodensonde hinsichtlich ihrer Kontaktfeder zur elektrischen Verbindung zwischen Gehäuse und Elektrodenwelle so zu verbessern, dass bei geringem Raumbedarf der Kontaktfeder eine zuverlässige Kontaktverbindung gewährleistet ist.

Diese Aufgabe wird durch die im Kennzeichnungsteil des Anspruches 1 angegebenen Merkmale gelöst. Demnach ist die Kontaktfeder als eine die Welle unter Federspannung umlaufende, im Wesentlichen ebene Spiralfeder ausgebildet, die mit ihren Schenkelenden unter Ausübung einer radial gerichteten Federkraft gleitend mit dem Gehäuse bzw. der Welle in Kontakt steht.

Durch die spiralige, um die Welle umlaufende Ausgestaltung der Kontaktfeder wird trotz kompakter Bauform die wirksame Federlänge erheblich vergrößert, wodurch eine flachere Federkennlinie bei harter Abstimmung der Feder erzielbar ist. Dies verleiht der Feder eine hohe Abstandstoleranz hinsichtlich sowohl einer axialen als auch rotatorischen Bewegung der Welle der Einschraubelektrode. Da ferner mindestens eines der Schenkelenden, vorzugsweise beide jeweils gleitend, also ohne feste Fixierung etwa in Form einer Verschweißung oder Verlötung mit dem Gehäuse bzw. der Welle in Kontakt steht, braucht die Kontaktfeder die rotatorische und axiale Verschiebung der Welle zum Einschrauben der Elektrode nicht mit zu machen. Sie braucht also diesbezüglich keinerlei Baulänge zur Verfügung stellen. Die damit mögliche, bereits oben erwähnte flache Federkennlinie zusammen mit der gegenüber dem Stand der Technik sehr großen Länge bei minimaler axialer Erstreckung gewährleistet gleichbleibende Kontaktkräfte mit geringer Schwankungsneigung auch bei einem aufgrund der Toleranzen im Elektrodenkopf regelmäßig stattfindenden Taumeln der Elektrodenwelle.

In den Unteransprüchen sind bevorzugte Weiterbildungen der Einschraubelektrodensonde im Hinblick auf die Kontaktfeder angegeben. Deren Merkmale, Einzelheiten und Vorteile ergeben sich aus der nachfolgenden Beschreibung eines Ausführungsbeispiels anhand der beigefügten Zeichnungen. Es zeigen:
- Fig. 1: einen schematischen Axialschnitt durch den Elektrodenkopf einer Einschraubelektrodensonde, und
- Fig. 2: einen Schnitt entlang der Schnittlinie II/II nach Fig. 1.

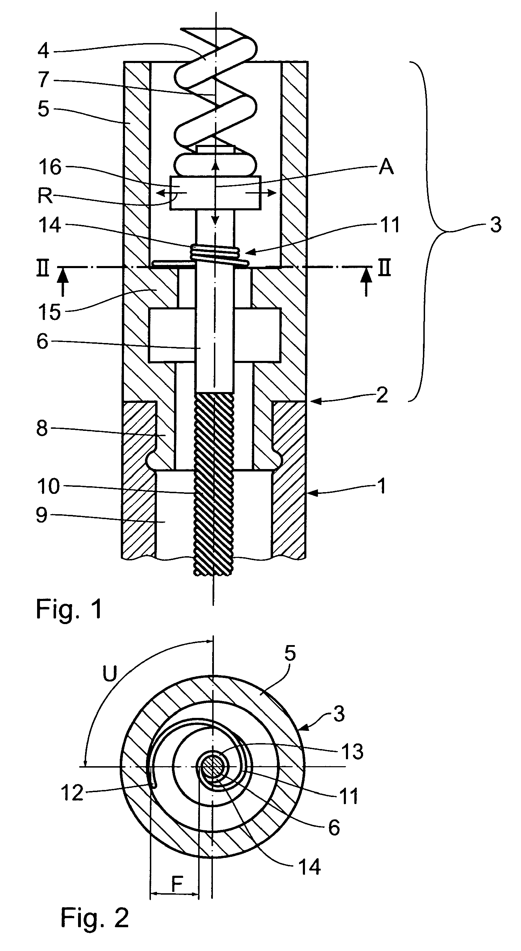

Wie insbesondere aus Fig. 1 deutlich wird, weist eine Einschraubelektrodensonde zur kardiologischen Anwendung einen langgestreckten, schlauchförmigen Elektrodenkörper 1 aus einem elektrisch isolierenden Material auf. Darin sind die verschiedenen Zuleitungen zu nicht näher dargestellten Elektroden, wie beispielsweise vom distalen Ende 2 der Sonde beabstandeten Ringelektroden untergebracht.

Am distalen Ende 2 des Elektrodenkörpers 1 selbst ist ein als Ganzes mit 3 bezeichneter Elektrodenkopf vorgesehen, der eine in kardialem Gewebe verankerbare, korkenzieherartige Einschraubelektrode 4 aufweist. Diese ist in einem im Wesentlichen zylindrischen Gehäuse 5 an einer Welle 6 um ihre Wendelachse 7 drehbar und in dieser Richtung axial verschiebbar gelagert. Zur Verbindung des Elektrodenkörpers 1 mit dem Gehäuse 5 ist eine Tülle 8 am Gehäuse 5 vorgesehen, auf die der Elektrodenkörper 1 mit seinem Lumen 9 aufgeschoben und dort in geeigneter Weise fixiert ist. In diesem Lumen 9 des Elektrodenkörpers 1 ist vom proximalen Ende der Elektrodensonde her eine Leitungswendel 10 herangeführt, die mit der Welle 6 mechanisch und in elektrischem Kontakt fest verbunden ist. Über die Leitungswendel 10 können elektrische Impulse zur Abgabe über die Einschraubelektrode 4 oder elektrokardiologische Messsignale von der Einschraubelektrode 4 geführt werden. Darüber hinaus ist die Leitungswendel 10 so torsionssteif, dass darüber ein Drehmoment vom proximalen Ende der Elektrodensonde her auf die Welle 6 zu deren Rotation um die Wendelachse 7 übertragen werden kann. Der axiale Vortrieb der Einschraubelektrode 4 wird dabei über einen sogenannten Steigungsgeber hervorgerufen, der in das Gehäuse 5 integriert, hier jedoch nicht eigens dargestellt ist.

Zur Herstellung eines elektrischen Kontaktes zwischen der Baueinheit aus Einschraubelektrode 4 und Welle 6 mit dem Gehäuse 5 ist eine Kontaktfeder vorgesehen, die als im Wesentlichen ebene Spiralfeder 11 ausgebildet ist. Die Erstreckungsebene dieser Spiralfeder 11 ist orthogonal zur Wendelachse 7 gerichtet, so dass sich die Schenkelenden 12, 13 der Spiralfeder 11 jeweils etwa auf gleicher Höhe einerseits an der Innenwand des Gehäuses 5 und andererseits an der Mantelfläche der Welle 6 abstützen. Dabei besteht keine starre Verbindung zwischen den Schenkelenden 12, 13 und den vorgenannten Bauteilen des Elektrodenkopfes, so dass zwischen Spiralfeder 11 und Welle 6 bzw. Gehäuse 5 ein gleitender Kontakt besteht. Wie aus Fig. 2 deutlich wird, ist die Spiralfeder 11 mit ihrer Schenkellänge so bemessen, dass sie unter radialer Spannung um die Welle 6 umläuft, wobei der Umschlingungswinkel U mindestens 360°, vorzugsweise jedoch - wie hier dargestellt - etwa 450° beträgt. Aufgrund der gezeigten und beschriebenen Anordnung übt die Spiralfeder 11 mit ihren beiden Schenkelenden 12, 13 eine Spreizkraft F aus, die für eine saubere Kontaktverbindung zwischen den damit verbundenen Elementen sorgt. Damit können sowohl die Axialverschiebung A der Einschraubelektrode 4 mit Welle 6 beim Verankern als auch dabei stattfindende Radialspiele R ausgeglichen werden ohne die Federkraft F und damit die Kontaktgabe zwischen den verbundenen Elementen zu beeinträchtigen.

Zur Unterstützung der Kontaktgabe zwischen Spiralfeder 11 und Welle 6 ist noch eine schraubenfederartige Verlängerung 14 am inneren Schenkelende 13 vorgesehen, die die Welle 6 unter Spiel mit zwei bis drei auf Block gewickelten Windungen umgreift. Im Übrigen sitzt die Spiralfeder 11 zwischen einem ringförmigen Absatz 15 an der Innenseite des Gehäuses 5 und einer Ringschulter 16 an der Welle 6. Auf dieser Ringschulter 16 sitzt auch die Einschraubelektrode 4 auf der der Spiralfeder 11 abgewandten Seite.

Die Kontaktfeder selbst besteht beispielsweise aus einem Federstahldraht. Bevorzugt besteht die Kontaktfeder aus Platin oder einer Platinlegierung. Die Platinlegierung enthält ungefähr 70 bis 80 % Platin, der Rest zu 100 % bildet sich aus Iridium. Der Durchmesser des Drahtes liegt zwischen 0,08 mm und 0,2 mm, besonders bevorzugt ist der Durchmesser des Drahtes 0,1 mm.

## Patentansprüche

1. Einschraubelektrodensonde zur kardiologischen Anwendung umfassend
- einen langgestreckten Elektrodenkörper (1) aus einem elektrisch isolierenden Material,
- eine im Elektrodenkörper (1) verlaufende, drehbare, elektrische Zuleitung (10), und
- einen Elektrodenkopf (3) am distalen Ende (2) des Elektrodenkörpers (1) mit
= einem Gehäuse (5),
= einer darin mit Hilfe der Zuleitung (10) drehbar und axial verschiebbar gelagerten, elektrisch leitfähigen Welle (6), die an der Zuleitung (10) in axialer Verlängerung angesetzt ist,
= einer korkenzieherartigen Einschraubelektrode (4) an der Welle (6), sowie
= einer zwischen Gehäuse (5) und Welle (6) eingesetzten Kontaktfeder (11) zu deren elektrischer Verbindung,
**dadurch gekennzeichnet, dass**
die Kontaktfeder als die Welle (6) unter Federspannung umlaufende, im wesentlichen ebene Spiralfeder (11) ausgebildet ist und mit ihren Schenkelenden (12, 13) unter Ausübung einer radial gerichteten Federkraft (F) gleitend mit dem Gehäuse (5) und/oder der Welle (6) in Kontakt steht.

2. Einschraubelektrodensonde nach Anspruch 1, **dadurch gekennzeichnet, dass** der Umschlingungswinkel (U) der Kontaktfeder (11) im eingesetzten Zustand um die Welle (6) mindestens 360°, vorzugsweise etwa 450° beträgt.

3. Einschraubelektrodensonde nach Anspruch 1 oder 2, **dadurch gekennzeichnet, dass** beide Schenkelenden (12, 13) jeweils gleitend mit dem Gehäuse (5) bzw. der Welle (6) verbunden sind.

4. Einschraubelektrodensonde nach einem der vorgenannten Ansprüche, **dadurch gekennzeichnet, dass** das der Welle (6) zugeordnete, innere Schenkelende (13) mit einer schraubenfederartigen Verlängerung (14) versehen ist, die um die Welle (6) herumgelegt ist.

5. Einschraubelektrodensonde nach Anspruch 4, **dadurch gekennzeichnet, dass** die Verlängerung (14) die Welle (6) unter Spiel umgreift.

6. Einschraubelektrodensonde nach einem der vorgenannten Ansprüche, **dadurch gekennzeichnet, dass** die Kontaktfeder (11) aus einem Federstahldraht besteht.

7. Einschraubelektrodensonde nach einem der Ansprüche 1 bis 5, **dadurch gekennzeichnet, dass** die Kontaktfeder (11) aus einem Platin- oder Platinlegierungsdraht besteht.

8. Einschraubelektrodensonde nach Anspruch 7, **dadurch gekennzeichnet, dass** die Platinlegierung ungefähr 70 bis 80 % Platin enthält und sich der Rest zu 100 % aus Iridium bildet.

9. Einschraubelektrode nach einem der Ansprüche 6 bis 8, **dadurch gekennzeichnet, dass** der Draht einen Durchmesser von 0,08 bis 0,2 mm, vorzugsweise 0,1 mm aufweist.
